# EUROPÄISCHE PATENTANMELDUNG

(11) **EP 1 062 933 A2**
(43) Veröffentlichungstag der Anmeldung: **27.12.2000**
(21) Anmeldenummer: 00110572.5
(22) Anmeldetag: 18.05.2000
(51) Int. Cl.: A61H 23/02, A61N 7/00

(54) **Vorrichtung, insbesondere Therapievorrichtung, zum Beschallen von Objekten mit fokussiertem Schall**

(30) Priorität: 22.06.1999 DE 19928491
(71) Anmelder: Richard Wolf GmbH, 75438 Knittlingen (DE)
(72) Erfinder: Bauer, Edgar, 76703 Kraichtal (DE); Krauss, Werner, 75438 Knittlingen (DE); Jaggy, Peter, 75443 Ötisheim (DE)
(74) Vertreter: Wilcken, Thomas, Dipl.-Ing.

(57) **Zusammenfassung**

Es geht um eine Vorrichtung, insbesondere eine Therapievorrichtung, mit der vor allem körperinnere Bereiche und Objekte mit fokussiertem Schall behandelt werden. Der Schall wird dabei von wenigstens einem elektroakustischen Wandler (1) erzeugt und an einer gekrümmten Abstrahlfläche (4) in Richtung auf eine gedachte Fokuslinie (5) abgestrahlt. Diese Abstrahlfläche (4) hat nur eine einzige definierte Symmetrieebene (6), in der die Fokuslinie (5) liegt.

## Beschreibung

Die Erfindung betrifft eine Vorrichtung, insbesondere Therapievorrichtung, zum Beschallen von Objekten mit fokussiertem Schall, der von mindestens einem elektroakustischen Wandler erzeugt und an einer gekrümmten Abstrahlfläche in Richtung auf eine gedachte Fokuslinie abgestrahlt wird.

Die extrakorporale Schalltherapie mit Druck- oder Stoßwellen wird erfolgreich in der Orthopädie zur nichtinvasiven Behandlung von knochennahen Weichteilen eingesetzt, und zwar beispielsweise bei den Indikationen Tennisellenbogen, Fersensporn und Kalkablagerungen im Schultergelenk. Bei diesen Behandlungen werden vorwiegend Sehnenansätze, Schmerzstellen oder Kalkdepots als Therapiezonen lokalisiert und mittels fokussiertem Schall in Form von Stoßwellen und/oder Druckwellen beschallt, wobei im allgemeinen 1000 bis 3000 Schallimpulse verteilt auf relativ kleine Therapiezonen innerhalb des Patientenkörpers appliziert werden.

Bei sonstigen Indikationen, wie etwa großflächige Muskelverspannungen, Muskelverhärtungen, Bindegewebsverdichtungen an den Extremitäten motorisch behinderter Patienten oder zur Behandlung von Cellulite (DE 197 21 218 A1) wünscht man sich eine homogene Dosierung der Schallenergie im gesamten zu therapierenden Bereich.

Dies könnte man mit einer Vorrichtung erreichen, die einen elektroakustischen Wandler aufweist, der automatisch und systematisch zum Beispiel in XY-Richtung über dem zu behandelnden Bereich verfahren wird. Eine solche Vorrichtung würde sich aber nur mit hohem mechanischen Aufwand realisieren lassen.

Insofern ist eine stationäre Multifokus-Vorrichtung weniger aufwendig, wenn diese durch mosaikartige Anordnung kleiner fokussierender Wandler so aufgebaut wird, daß die Fokusse der Wandler in einer Ebene oder auf einer gedachten Linie liegen, die in Überdeckung mit der Therapiezone im Patientenkörper gebracht werden kann. Eine solche Fokuslinie könnte ein Kreis sein (DE 39 32 967 C2), dessen Durchmesser allerdings relativ klein sein wird, weil sonst der bauliche Aufwand für eine solche Vorrichtung nicht akzeptabel wäre, abgesehen davon, daß zum Betrieb einer derartigen Vorrichtung viel Energie erforderlich wäre und die Schallabstrahlfläche insgesamt extrem groß sein müßte, wenn man großflächig und gleichmäßig mit der erforderlichen Schallenergie therapieren wollte, was im übrigen auch noch den Nachteil mit sich bringen würde, daß insbesondere beim Beschallen hautnaher Therapiezonen der Patient Schmerzen erleiden könnte.

Jedenfalls lassen sich in der Praxis mit bisher bekannten Vorrichtungen großflächige Gewebebereiche und Therapiezonen nur sukzessiv mit relativ kleinen akustischen Behandlungszonen mit Ausdehnungen in der Größenordnung von 2 bis 10 mm wandernd abtasten. Diese Art Behandlung ist sehr zeitintensiv. Außerdem wird eine über die vorgesehene Therapiezone im Patientenkörper homogene Applikation des Schalls nicht erreicht, wenn der Wandler üblicherweise an einem Gelenkarm angebracht ist und durch manuelles Verstellen des Gelenkarms der Wandler über oder auf der Hautoberfläche des Patienten verschoben werden muß.

Die aufgezeigten Nachteile sollen durch die Erfindung mit einer preisgünstigen und einfach zu bedienenden Vorrichtung beseitigt werden, mit der es möglich ist, insbesondere auch großflächige Therapiezonen schnell, gleichmäßig bzw. homogen und effektiv mit Schall zu behandeln.

Diese Aufgabe wird bei einer gattungsgemäßen Vorrichtung der eingangs erwähnten Art so gelöst, daß die Abstrahlfläche eine einzige Symmetrieebene hat und daß die Fokuslinie in dieser Symmetrieebene liegt. Eine relativ einfache Geometrie der Abstrahlfläche kann man erreichen, wenn diese die Form einer Teilmantelfläche eines Kreiszylinders hat und der Schall auf die die Fokuslinie bildende Mittelachse des Kreiszylinders fokussiert wird.

Wenn der Wandler selbstfokussierend ausgebildet wird, bildet die der Fokuslinie zugewandte Wandlerfläche die Abstrahlfläche. Als Wandler kommt sonst auch ein mindestens mit einer akustischen Linse ausgestatteter Planarwandler in Betracht, wobei dann die der Fokuslinie zugewandte Linsenoberfläche die Abstrahlfläche bildet.

Weitere vorteilhafte Merkmale der erfindungsgemäßen Vorrichtung sind in Unteransprüchen angegeben.

In der Zeichnung sind einige Ausführungsbeispiele für nach der Erfindung ausgebildete Vorrichtungen und Vorrichtungsteile schematisch und vereinfacht dargestellt. Es zeigt:
- Fig. 1: perspektivisch einen selbstfokussierenden Wandler,
- Fig. 2: den Wandler nach Fig. 1 im Querschnitt mit weiterer Ausstattung,
- Fig. 3: den Wandler gemäß den Fig. 1 und 2 in Verbindung mit einem B-Scanner einer Ortungseinrichtung,
- Fig. 4: einen zur Ortungseinrichtung gehörenden Monitor,
- Fig. 5: eine Vorrichtung mit mehreren eine Kette bildenden Wandlern,
- Fig. 6: eine erfindungsgemäße Vorrichtung im Querschnitt,
- Fig. 7: perspektivisch eine Ausführungsform für einen mit einer akustischen Linse ausgestatteten Planarwandler,
- Fig. 8: perspektivisch eine weitere Ausführungsform für einen Planarwandler mit akustischer Linse,
- Fig. 9: einen in fokussierende Segmente unterteilten Wandler in Draufsicht und
- Fig. 10: ein Spannungs-Zeit-Diagramm elektrischer Impulse zur Ansteuerung der Wandlersegmente nach Fig. 9.

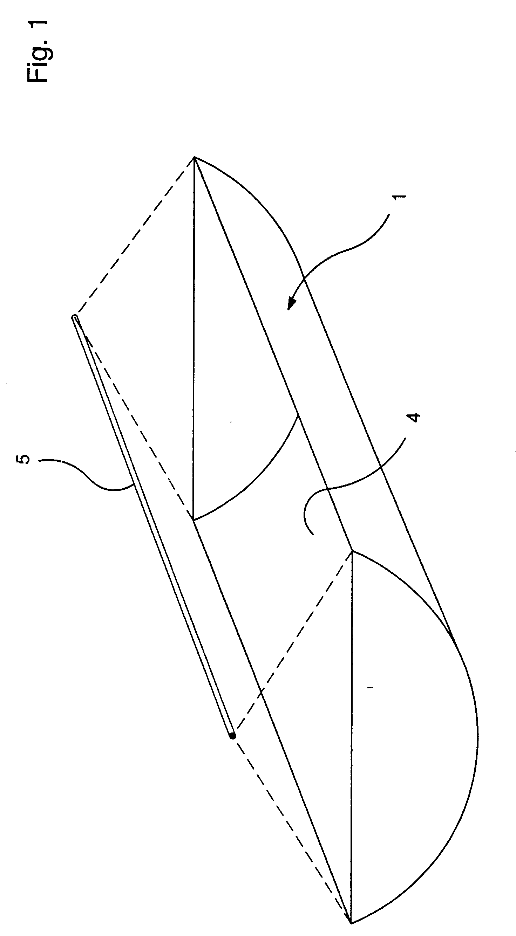

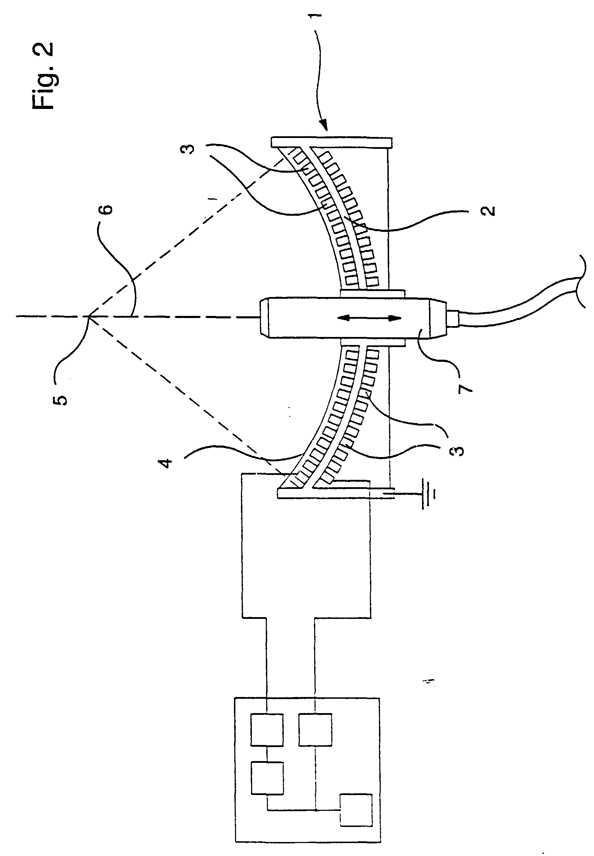

Bei dem in den Fig. 1 bis 3 dargestellten Wandler 1 ist ein Träger 2 vorgesehen, der beidseitig mosaikartig mit piezoelektrischen Elementen 3 (Piezoelemente) bestückt ist (Fig. 2). Diese Bauweise, deren Vorteile und auch die Funktion und Steuerung solcher Wandler sind grundsätzlich bekannt (DE 197 33 233 C1) und werden deshalb nicht im einzelnen beschrieben.

Anders als bei bekannten Wandlern mit einem Träger in Form einer Kugelkalotte hat der trogartige Träger 2 die Form eines Längsabschnittes eines Kreiszylinders, so daß zwangsläufig auch die Abstrahlfläche 4 des Wandlers die Form einer Teilmanteifläche eines in diesem Fall geraden Kreiszylinders hat und der Schall in Richtung auf eine Fokuslinie 5 abgestrahlt sowie auf diese Linie fokussiert wird, auf welche die Piezoelemente mit ihren Achsen ausgerichtet sind.

Die Fokuslinie 5 stimmt mit der Mittelachse des erwähnten und gedachten Kreiszylinders bzw. Zylinderabschnitts überein. Im übrigen hat dann die Abstrahlfläche 4, die beispielsweise durch die zur Fokuslinie gerichtete Oberfläche einer die Piezoelemente abdeckenden Metallmembran gebildet wird, bei dieser Bauform zwangsläufig nur eine Symmetrieebene 6, in der die Fokuslinie 5 liegt, deren Länge durch die ausgewählte Länge des Wandlers 1 bestimmt wird. Hiermit wird auch festgelegt, über welche Breite die Therapiezone beim Bewegen der Vorrichtung bzw. des Wandlers jeweils erfaßt werden kann.

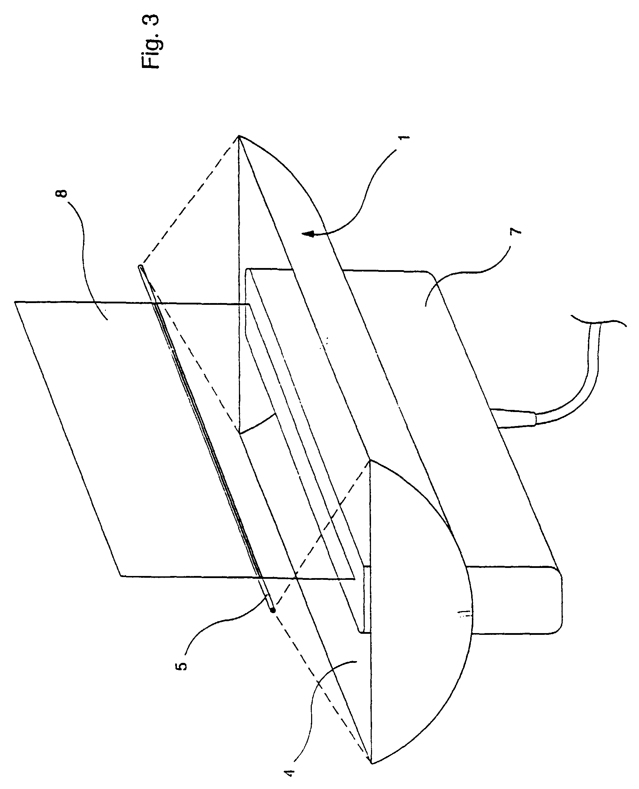

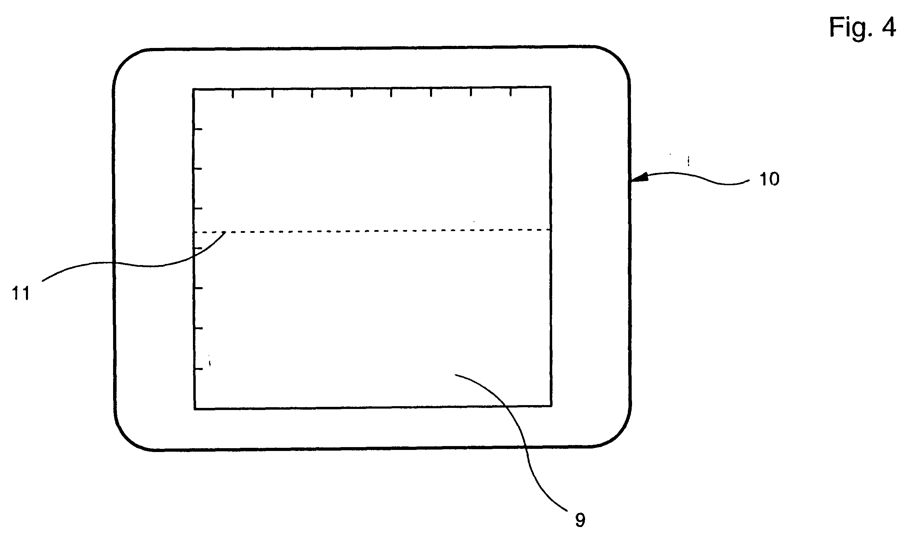

Zum Lokalisieren des zu therapierenden Bereiches kann ein dem Wandler 1 extern zugeordneter Linearschallkopf eines Ultraschall-B-Scanners verwendet werden. Zur Erzielung einer kompakten Bauform wird aber vorzugsweise ein B-Scanner 7 mit seinem Linearschallkopf innerhalb des Wandlers 1 gemäß den Fig. 2 und 3 inline angeordnet, und zwar im Zentrum des Wandlers in der Symmetrieebene 6 der Abstrahlfläche 4 linear nach oben und unten verschiebbar, so daß die Ultraschallschnittfläche 8 durch die Fokuslinie 5 verläuft. Im Ultraschallschnittbild 9 auf einem Monitor 10 wird eine Therapielinie 11 dargestellt (Fig. 4), welche die Fokuslinie 5 repräsentiert. Bei Verschiebung des Linearkopfes des B-Scanners wird die Therapielinie 11 entsprechend den jeweiligen Verschiebestrecken auf dem Monitorbild je nach Verschieberichtung nach oben oder unten automatisch nachgeführt.

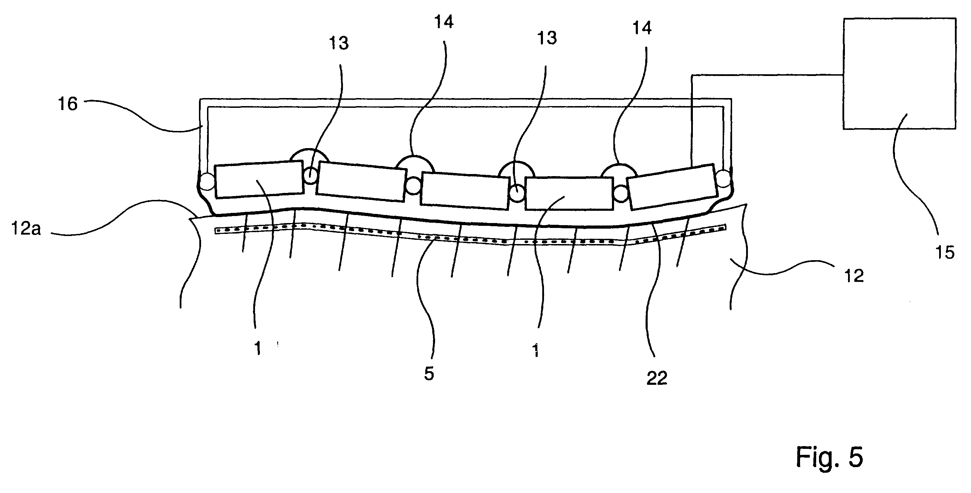

Durch Aneinanderreihen mehrerer beweglich miteinander verbundener Wandler 1, gemäß Fig. 5 können dies beispielsweise fünf Wandler sein, kann eine Wandlerkette gebildet werden, mit der sich eine Fokuslinie erzeugen läßt, deren Länge sich bei Vernachlässigung der Abstände zwischen benachbarten Wandlern aus der Summe der Fokuslinien der einzelnen Wandler ergibt. Außerdem werden sich bei einer so aufgebauten Therapievorrichtung die Fokuslinie 5 und auch der Verlauf der Wandlerkette den anatomischen Gegebeneinheiten auf der Oberfläche eines Patientenkörpers 12 anpassen.

Die Wandler sind mit Scharnieren, flexiblen Elementen 13 oder dergleichen und auch über Kabel 14 miteinander verbunden und werden gleichzeitig mittels einer Steuerung 15 zur Abgabe von Stoßwellen oder Druckwellen aktiviert. Die beiden jeweils in der Wandlerkette äußeren Wandler sind an einem starren Bügel 16 so angelenkt, daß alle Wandler zwar im wesentlichen als Kette ausgerichtet bleiben, aber doch relativ zueinander geneigte Positionen gemäß Fig. 5 einnehmen können, wenn die Therapievorrichtung auf der Hautoberfläche 12a des Patienten in quer zur Wandlerkette liegender Richtung bewegt wird, um dabei mit der Fokuslinie die vorab lokalisierte Therapiezone zu behandeln.

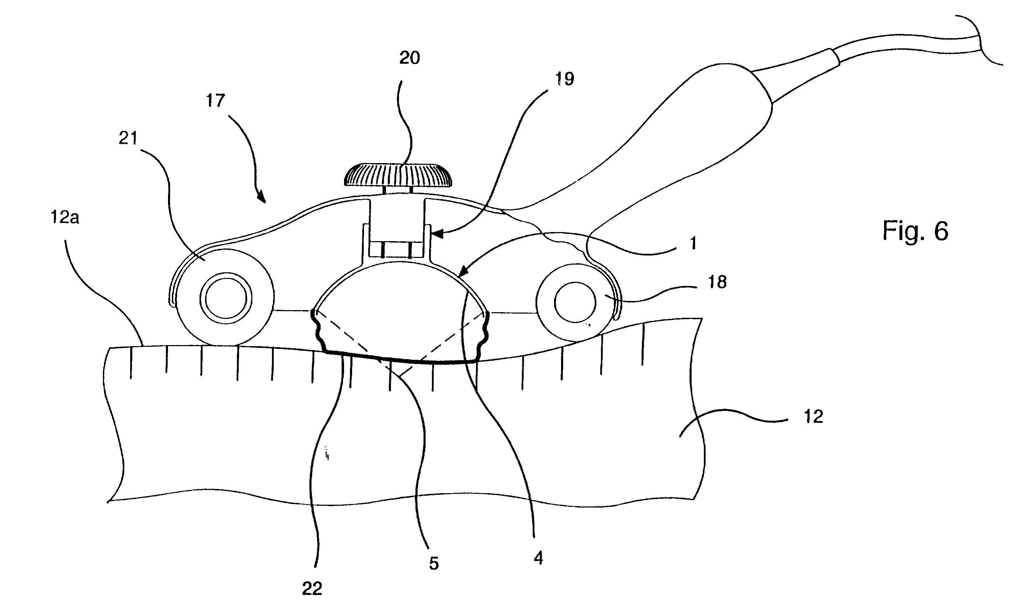

Eine besondere Ausführungsform zur Behandlung bzw. Stimulation von subkutanen Muskel-, Fett- und Bindegewebsbereichen, z. B. Cellulitegewebe sowie Gefäße (Lymphe, Venen und Arterien) oder Nervenzellen ist in Fig. 6 dargestellt. Dieses Therapiegerät 17 besteht aus einem Wandler 1 der vorher oder später in Verbindung mit weiteren Figuren beschriebenen Art. Das Gerät läßt sich auf Rollen 8 auf der Haut des Patientenkörpers 12 leicht manuell bewegen.

Der Wandler 1 hängt an einer Verstelleinrichtung 19 mit einer Handhabe 20, mit welcher der Abstand des Wandlers 1 und damit seine Abstrahlfläche 4 zur Haut- bzw. Bewegungsfläche 12a auf dem Patientenkörper eingestellt werden kann, um so die Eindringtiefe der Fokuslinie 5 in den Körper variieren und an die Tiefe der mit Schall zu behandelnden Therapiezone anpassen zu können. Über einen Gel-Dispenser 21 wird während des Verschiebens des Geräts die Haut des Patienten mit Gel als akustisches Koppelmedium benetzt, so daß eine luftblasenfreie akustische Kopplung zwischen der Haut und einer zum Wandler 1 gehörenden Koppelmembran 22 erreicht wird.

Dieses Therapiegerät stellt eine preisgünstige Lösung dar und ist auch in Kliniken und Arztpraxen für eine ambulante Behandlung zu verwenden. Speziell für den erwähnten Einsatzbereich der Cellulite-Behandlung ist das Gerät als kleiner handlicher Applikator herstellbar, der sich ohne Mühe manuell auf der Haut oberhalb des Therapiebereiches vor- und zurückschieben läßt. Die Behandlung kann auch vom Patienten selbst ohne ärztliche Aufsicht durchgeführt werden, wobei erforderlichenfalls die Energie des vom Wandler abgestrahlten Schalls reduziert werden könnte.

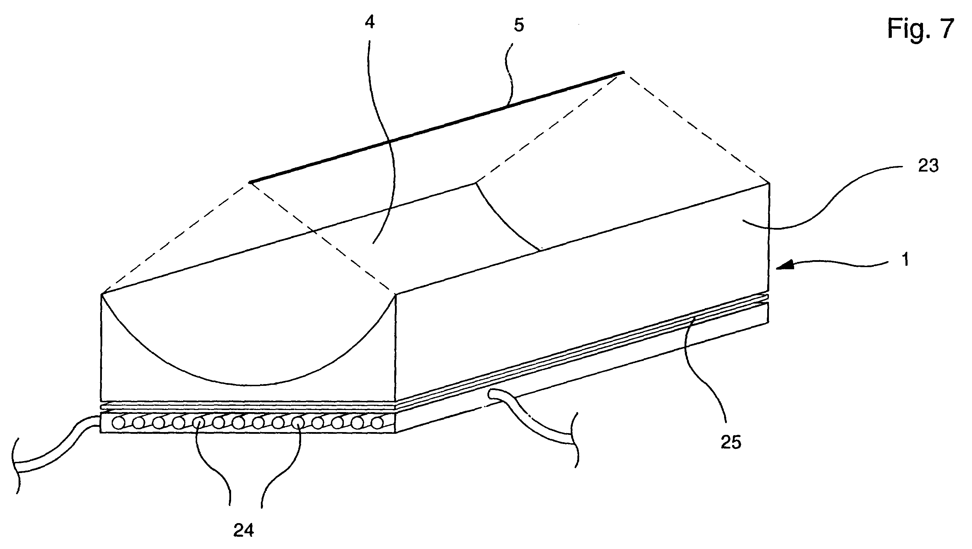

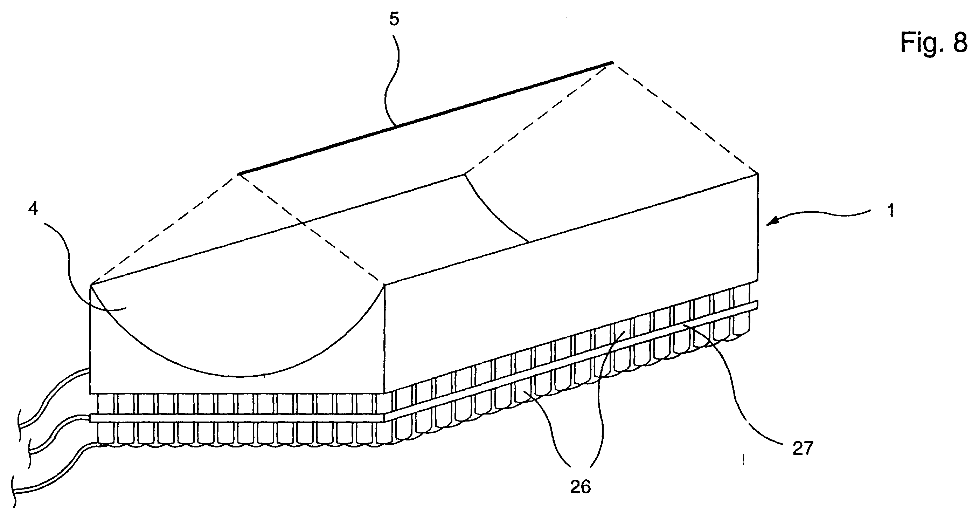

In den Fig. 7 und 8 sind jeweils planare elektroakustische Wandler 1 gezeigt, die mit akustischen Linsen 23 ausgestattet sind, um den Schall auf die Fokuslinie 5 zu richten und zu fokussieren. In diesen Fällen bilden die der Fokuslinie 5 zugewandten Oberflächen der Linsen 23 die Abstrahlflächen, die im Querschnitt gesehen parabelförmig gekrümmt verlaufen und ebenfalls nur eine einzige Symmetrieebene haben.

Im übrigen handelt es sich beim Wandler 1 nach Fig. 7 um einen elektromagnetischen Planarwandler mit einer Spule 24 und einer Metallmembran 25 und beim Wandler 1 nach Fig. 8 um einen piezoelektrischen Planarwandler mit einer auf beiden Seiten mit Piezoelementen 26 bestückten Kontaktierungsplatte 27 aus Metall. In dieser Bauweise und auch in der Funktion stimmen diese Wandler im Prinzip mit bekannten Planarwandlern überein, bei denen über eine Linse der Schall auf nur einen Punkt fokussiert wird.

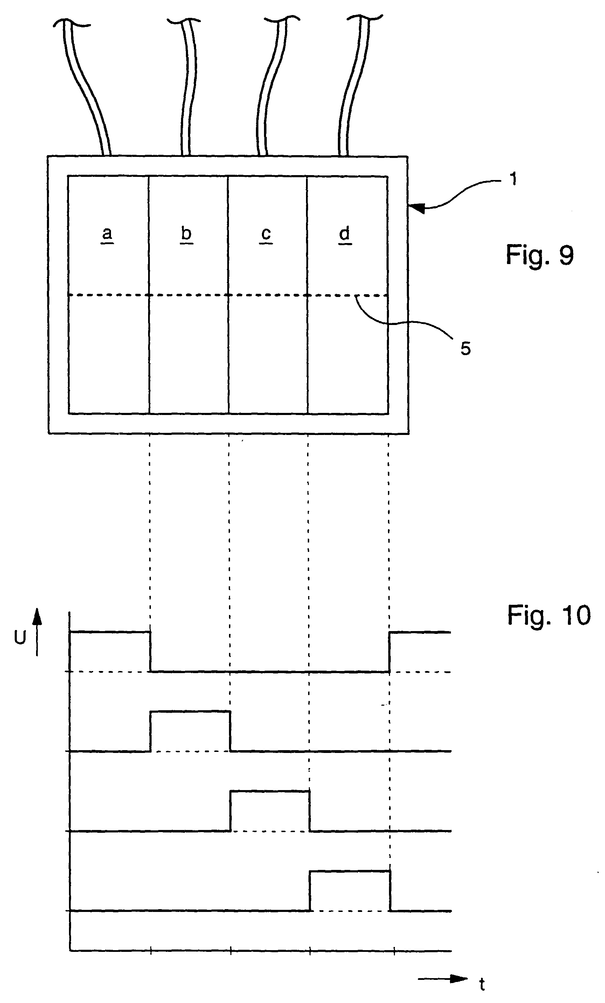

Der Wandler gemäß Fig. 9 ist hinsichtlich seines Schall abgebenden Teils in vier Abschnitte bzw. Segmente a, b, c und d unterteilt, die beispielsweise in der Reihenfolge a, b, c und d zur Schallerzeugung zeitlich versetzt angesteuert werden können und den Schall auf die Fokuslinie 5 richten und fokussieren. Die für diese Betriebsweise notwendigen Steuerimpulse ergeben sich aus dem in Fig. 10 gezeigten Diagramm, bei dem der Verlauf der Spannung U dieser Impulse über der Zeit t eingezeichnet ist. Durch die von der Fig. 10 nach oben geführten gestrichelten Linien soll nur deutlich gemacht werden, mit welchen Impulsen welche Wandlersegmente zeitlich versetzt angesteuert werden. Eine andere Anzahl und Größe der Wandlersegmente, eine andere Reihenfolge für die Ansteuerung der Wandlersegmente, eine zeitverzögerte Ansteuerung von Wandlersegmentgruppen und auch zeitlich andere Ansteuerungsverzögerungen sind ebenfalls möglich.

Im Zusammenhang mit der Fig. 5 wurde eine Kette aus aneinandergereihten Wandlern 1 beschrieben. Natürlich besteht auch die Möglichkeit, zwei oder mehr Wandler so anzuordnen, daß sie und ihre Fokuslinien parallel zueinander verlaufen, was vorteilhaft wäre, wenn etwa nur relativ kleinflächige Therapiezonen effektiv behandelt werden sollen.

Im Zusammenhang mit dem Begriff "Fokuslinie" ist noch anzumerken, daß es sich in der Praxis nicht ideal erreichen lassen wird, den Schall auf eine Linie ohne räumliche Ausdehnung zu fokussieren. Das ist auch nicht anders als bei bekannten fokussierenden Wandlern, bei denen man auch nur anstreben kann, den Schall auf einen Fokuspunkt zu konzentrieren. In entsprechender Weise ist sinngemäß die gedachte "Fokuslinie" zu verstehen, die übrigens auch in einer einzigen Symmetrieebene einer im Raum gekrümmten Abstrahlfläche liegen kann.

Wie Versuche gezeigt haben, ist die erfindungsgemäße Vorrichtung neben technischen Einsatzgebieten auch auf dem Gebiet der Lithotripsie einsetzbar, bei der dann körperinnere Konkremente auf der Oberfläche entlang der Fokuslinie und durch deren Bewegung auf oder im Konkrement nach und nach abgetragen werden.

## Patentansprüche

1. Vorrichtung, insbesondere Therapievorrichtung, zum Beschallen von Objekten mit fokussiertem Schall, der von mindestens einem elektroakustischen Wandler (1) erzeugt und an einer gekrümmten Abstrahlfläche (4) in Richtung auf eine gedachte Fokuslinie (5) abgestrahlt wird, dadurch gekennzeichnet, daß die Abstrahlfläche (4) eine einzige Symmetrieebene (6) hat und daß die Fokuslinie (5) in dieser Symmetrieebene liegt.

2. Vorrichtung nach Anspruch 1, dadurch gekennzeichnet, daß die Abstrahlfläche (4) die Form einer Teilmantelfläche eines Kreiszylinders hat und daß der Schall auf die die Fokuslinie (5) bildende Mittelachse des Kreiszylinders fokussiert wird.

3. Vorrichtung nach einem der Ansprüche 1 und 2, dadurch gekennzeichnet, daß der Wandler (1) selbstfokussierend ist und daß die der Fokuslinie (5) zugewandte Wandlerfläche die Abstrahlfläche (4) bildet.

4. Vorrichtung nach einem der Ansprüche 1 und 2, dadurch gekennzeichnet, daß der Wandler (1) ein mit mindestens einer akustischen Linse (23) ausgestatteter Planarwandler ist und daß die der Fokuslinie (5) zugewandte Linsenoberfläche die Abstrahlfläche (4) bildet.

5. Vorrichtung nach einem der Ansprüche 1 bis 4, dadurch gekennzeichnet, daß am Wandler (1) ein in seiner Symmetrieebene (6) senkrecht in bezug auf die Fokuslinie (5) verstellbarer B-Scanner (7) mit linearem Schallabstrahlkopf vorgesehen ist.

6. Vorrichtung nach einem der Ansprüche 1 bis 5, dadurch gekennzeichnet, daß der Wandler (1) manuell auf einer Fläche (12a) bewegbar ist.

7. Vorrichtung nach Anspruch 6, dadurch gekennzeichnet, daß der Abstand zwischen der Bewegungsfläche (12a) und der Abstrahlfläche (4) einstellbar ist.

8. Vorrichtung nach einem der Ansprüche 1 bis 7, dadurch gekennzeichnet, daß der Wandler (1) mit einer Koppelmembran (22) ausgestattet ist.

9. Vorrichtung nach Anspruch 8 als Therapiegerät (17) zum Beschallen von körperinneren Objekten, insbesondere von Gewebe, Steinen und Ablagerungen, gekennzeichnet durch einen Gel-Dispenser (21) zum Benetzen der Hautoberfläche (12a) eines Patienten mit Gel als akustisches Koppelmedium zwischen der Hautoberfläche und der Koppelmembran (22).

10. Wandler nach einem der Ansprüche 1 bis 9, dadurch gekennzeichnet, daß der Wandler (1) in mehrere Segmente (a bis d) unterteilt ist, die wahlweise einzeln und in Gruppen zeitversetzt zur Schallerzeugung ansteuerbar sind.

11. Vorrichtung nach einem der Ansprüche 1 bis 10, dadurch gekennzeichnet, daß mehrere beweglich miteinander verbundene Wandler (1) eine Wandlerkette bilden.
